# EUROPEAN PATENT APPLICATION

(11) **EP 2 821 388 A1**
(43) Date of publication of application: **07.01.2015**
(21) Application number: 13175419.4
(22) Date of filing: 05.07.2013
(51) Int. Cl.: C07C 201/12

(54) **Fluorination process**

(71) Applicant: Solvay SA, 1120 Bruxelles (BE)
(72) Inventor: Jaunzems, Janis, 30559 Hannover (DE); Claassen, Uta, 31249 HOHENHAMELN (DE); Braun, Max Josef, 30900 WEDEMARK (DE)
(74) Representative: Mross, Stefan P.M.

(57) **Abstract**

It has been found that a compound of formula (I) R-CF₂-O-Ar, wherein R is a fluorinated alkyl group or an optionally substituted aromatic or heteroaromatic group and Ar is an optionally substituted aromatic or heteroaromatic group, can be produced by a process comprising a step of reacting a compound of formula (II) R-C(O)-O-Ar wherein R and Ar are as defined above with a fluorination agent at a temperature of below +100°C.

## Description

Aromatic perfluorethers have a variety of industrial application, such as use in plant-protecting and pharmaceutical compositions (e.g. US4824863).

Sheppard et al (Journal of Organic Chemistry (1964), 29(1), 1-11) describes the reaction of sulphur tetrafluoride with aryl fluoroformates or perfluoralkyl esters to provide a synthesis of aryl perfluoroalkyl ethers. The reaction conditions are harsh with high temperatures; the paper states that "At temperatures below 150°C, the carbonyl group reacted only to a small extend, and the major portion of the fluoroformate or ester was recovered" (page 2, 2nd column). The Sheppard reference discloses reaction temperatures of 100°C, 150°C and 175°C (see method C, page 9).

US4695657 claims a process for esterification of aromatic alcohols with acid chlorides and simultaneous fluorination of the resulting ester to form a difluoromethyl group α to the oxygen of the intermediary ester. The process is carried out in the presence of BF₃. The examples show poor selectivity; none of the desired products was isolated in pure form.

It has surprisingly been found that a compound of formula (I) R-CF₂-O-Ar, wherein R is a fluorinated alkyl group or an optionally substituted aromatic or heteroaromatic group and Ar is an optionally substituted aromatic or heteroaromatic group, can be produced by a process comprising a step of reacting a compound of formula (II) R-C(O)-O-Ar wherein R and Ar are as defined above with a fluorination agent at a temperature of below +100°C. The process allows for high selectivity and yields and reduced energy consumption, which makes the process environmentally beneficial. The process allows the utilization of starting materials (e.g. trifluoroacetic acid chloride) which are available in industrial scale.

The term "fluorinated alkyl group" is intended to denote in particular a linear or branched alkyl substituent comprising from 1 to 10, preferably from 1 to 4, carbon atoms, wherein at least one carbon atom in the alkyl group is substituted by at least one fluorine atom. The fluorinated alkyl group is described by the formula CₘX₍₂ₘ₊₁₋ₙ₎Fₙ, wherein m is chosen from 1 to ten, n = 1-2m+1 and X is H or a substituent, wherein the substitutent is preferably Cl. m is preferably chosen from 1 to 4.

In a preferred embodiment, the fluorinated alkyl group R is CF₃, CHF₂, CH₂F, CClF₂, CHClF or CCl₂F.

In a more preferred embodiment, the fluorinated alkyl group R is CF₃, CClF₂ or CCl₂F.

In a most preferred embodiment, the fluorinated alkyl group R is CF₃.

The term "aromatic group" is intended to denote an aromatic group comprising from 6 to 24 carbon atoms, preferably from 6 to 12 carbon atoms. Specific examples of such groups are phenyl, 1-naphtyl and 2-naphtyl. A preferred aromatic group is phenyl.

The aromatic group can optionally be substituted by one or more substituents like an alkyl group or an electron withdrawing group. Specific examples of aromatic groups substituted by alkyl are 1-tolyl, 2-tolyl, 3-tolyl, xylyl. Preferably, the substituents of the aromatic group have electron withdrawing properties. More preferably, the substituents are chosen from F, Cl, I, Br, NO₂, CN. In an even more preferred embodiment, the aromatic group is substituted with NO₂. In the most preferred embodiment, Ar is p-NO₂-Ph.

The term "heteroaromatic group" is intended to denote an aromatic system having from 1 to 14 annular carbon atoms and at least one annular nitrogen, oxygen or sulfur atom such as furan, benzofuran, isobenzofuran, pyrrole, indole, isoindole, thiophene, benzothiophene, benzo[c]thiophene, imidazole, benzimidazole, purine, pyrazole, indazole, oxazole, benzoxazole, thiazole, benzothiazole, tetrazole, pyridine, quinoline, isoquinoline, pyrazine, quinoxaline, acridine, pyrimidine, quinazoline and pyridazine.

The heteroaromatic group can optionally be substituted by one or more substituents like an alkyl group or an electron withdrawing group.

The term "fluorinating agent" is intended to denote a reagent which is capable of fluorinating the carbonyl group of a compound of formula (II) R-C(O)-O-Ar. Specifically, the fluorinating agent is capable of fluorinating the carbonyl group of a compound of formula (II) R-C(O)-O-Ar at a temperature of below +100°C. In a preferred embodiment the fluorination agent is a compound comprising a S-F-bond or a B-F-bond. Specific examples of such a fluorinating agent are BF₃ and SF₄. In a most preferred embodiment, the fluorinating agent is SF₄.

In one embodiment of the invention, the process is conducted in the presence of a fluorination catalyst chosen from the group consisting of inorganic fluorides, preferably HF, AsF₃, PF₅ or TiF₄. It has been found in the process according to the present invention that these fluorination catalysts, specifically HF, do not fluorinate carbonyl groups, especially ester carbonyl groups, at temperatures of below +100°C, but they catalyze the reaction of fluorinating agents in the sense of the present invention The aromatic group can optionally be substituted by one or more substituents like an alkyl group or an electron withdrawing group. Without wishing to be bound by any particular theory, it is believed that the fluorination catalysts coordinate with the carbonyl oxygen of the compound of formula (II), thereby polarizing the carbonyl-oxygen-bond.

In a most preferred embodiment, the fluorination catalyst selected for the process of the present invention is HF.

In the process utilizing a fluorination catalyst, the molar ratio of fluorination catalyst to compound (II) is generally below 1. Preferably, the molar ratio of fluorination catalyst to compound (II) is from 0.6:1 to 0.01:1, more preferably from 0.4:1 to 0.05:1 and most preferably about 0.1:1.

In one embodiment of the invention, the fluorination process is generally carried out without adding a solvent.

In another embodiment, the fluorination process is generally carried out with the addition of a solvent. If appropriate, solvents can be chosen from ..., or, if suitable, an excess of reagent. This is especially advantageous when the reagent which is used as solvent is volatile at ambient pressure and temperature, or vacuum and/or heating. In a most preferred embodiment, HF is used as a solvent.

When HF is used as a solvent, the initial molar ratio of HF to compound of formula (II) is generally from 2:1 to 6:1. The most preferred initial molar ratio is about 4:1.

It has surprisingly been found that the conversion of a compound of formula (II) R-C(O)-O-Ar to a compound of formula (I) R-CF₂-O-Ar proceeds readily at temperatures of below +100°C. Generally, the process is carried out at a temperature equal to or below 90°C, preferably at a temperature of equal to or below +60°C, more preferably at a temperature of equal to or below +40°C. Generally, the process is carried out at a temperature equal to or greater than 0°C, preferably at a temperature of equal to or greater than +10°C, more preferably at a temperature of equal to or greater than +20°C. A temperature of about 25°C is more particularly preferred.

In one aspect of the process according to the invention, the compound of formula (I) can be recovered from the reaction medium by removing in the gaseous phase components of the reaction medium which are more volatile than the compound of formula (I) such as e.g. reagents and catalysts. Examples of such reagents and catalysts are HF and SF₄. This can be carried out for example by applying a vacuum and/or heating. It has been found that generally this isolation procedure alone allows to recover compound of formula (I) in satisfactory purity for further use.

In another aspect, isolation may also comprise any combination of filtration, washing, drying, distillation and/or cystallization steps.

In another embodiment, the process further comprises a step 1a wherein the compound of formula (II) R-C(O)-O-Ar is manufactured by reacting a compound of formula (III) R-C(O)Cl with a compound of formula (IV) Ar-OH, wherein Ar and R are defined as above. This step 1a is preferably performed in the presence of an acid scavenging agent. In this case, the molar ratio of acid scavenging agent to compound of the formula (IV) is generally below 1. The molar ratio of acid scavenging agents to compound (IV) of 1:1 or even a molar excess of acid scavenging agent to compound (IV) is not needed as the onium compound formed by the acid scavenging agent and evolving acid effectively catalyzes the reaction. In a preferred embodiment, the acid scavenging agent is added in step 1a in a molar ratio of acid scavenging agent to compound (IV) of below 1:1. In a more preferred embodiment, the molar ratio is between 0.001:1 and 0.8:1. In an even more preferred embodiment, the molar ratio is between 0.001:1 and 0.5:1. The most preferred molar ratio is chosen between 0.004:1 and 0.02:1.

The term "acid scavenging agent" is intended to denote a compound which is capable of reacting with an acid in particular HCl, formed during the reaction of compound (III) with compound (IV). In a preferred embodiment, this acid scavenging agent is an amine of the formula NR'R"R"', wherein R', R" and R"' are, independently of one another, hydrogen, alkyl having from 1 to 20 carbon atoms, aryl or aralkyl, or where R' and R", or where R' and R"', or where R" and R'" or where R', R" and R"' form saturated or unsaturated ring systems, optionally with inclusion of the nitrogen atom. In this context the term "aryl" refers in particular to phenyl or phenyl substituted by one or more C1-C2-alkyl groups. Acid scavenging agent which are suitable include ammonium, pyridine, piperidine, aniline, benzyltriethylamine and triethylamine. In a most preferred embodiment, the acid scavenging agent is pyridine.

Without wanting to be bound by theory, it is believed that the amine acid scavenging agents form an onium salt with evolving acid, in particular HCl in step 1a and that the onium salt catalyzes the reaction between compound of formula (III) and compound of formula (IV). The term "onium" is intended to denote a molecule with a positively charged nitrogen atom. The onium salt catalyst is usually selected from salts formed between the cation of the acid scavenging agent as defined above, and anions chosen from halide anions or the anion of the corresponding acid of compound (III), denoted by formula (V) R-C(O)O⁻, wherein R is defined as for R in compound (I) as stated above.

Additional formed acid, if in gaseous state, can be advantageously withdrawn from the reaction mixture as a gas.

The onium salt may also be provided to the reaction mixture, by addition of an onium salt catalyst as described above. The onium salt may be formed in situ or in a further reaction step preceding step 1a, for example by reacting a halide salt of the acid scavenging agent as defined above with the corresponding anhydride of compound (III), a compound (VI) (R-C(O))₂O, wherein R is defined as for R in compound (I) as stated above. In another embodiment, the onium salt is added to step 1a in isolated form. In this case the reaction between the compound of formula (III) and the compound of formula (IV) may be suitably carried out in the absence of the acid scavenging agent.

In another preferred embodiment, the onium salt of the acid scavenging agent as defined above is added to the reaction mixture, or formed in situ before step 1a, and no further acid scavenging agent is added to this step. In this preferred embodiment, the preferred molar ratio of onium salt to compound (IV) of below 1:1. In a more preferred embodiment, the molar ratio is from 0.001:1 to 0.8:1. In an even more preferred embodiment, the molar ratio is from 0.001:1 to 0.5:1. The most preferred molar ratio is chosen between 0.004:1 and 0.02:1.

Step 1a of reacting compound (III) with compound (IV) generally proceeds quantitatively and selectively. The reaction may be performed with or without adding a solvent. Suitable solvents are chosen form the group of polar aprotic solvents. Examples of polar aprotic solvents include esters like ethyl acetate, amides like dimethyl formamide, alkyl ethers like tetrahydrofurane and diethyl ether and nitriles like acetonitrile. In a preferred embodiment, the reaction of step 1a is carried out in diethylether or dimethoxyethane. The product (II) can be advantageously recovered by filtering off the onium salt and removing the solvent, if used, and any volatiles in a vacuum. Generally, the purity of (II) after filtration and removal of volatiles is >95 %.

A preferred process according to the current invention comprises the consecutive steps of (a) reaction of compound (III) with compound (IV) (step 1a), (b) purification of (II) by filtration and evaporation and (c) fluorination of compound (II) to yield compound (I).

In another aspect, the crude product (II) from step 1a may be subjected to purification methods, e.g. washing steps, extraction steps, drying steps, distillation, crystallization and the like, before further being fluorinated to compound (I).

In one embodiment, the reaction temperature of step 1a is from -78°C to +90°C. In a more preferred embodiment, the reaction temperature of step 1a is from -20°C to 70°C. Most preferably, the reaction temperature of step 1a is from 0°C to 65°C.

### Examples

### Example 1 : Synthesis of 4-nitrophenyl 2,2,2-trifluoroacetate

5g (36mmol) p-nitrophenol was dissolved in 10 ml dimethoxyethane and 0.05 ml (0.6 mmol) pyridine was added. The flask was equipped with a dry ice condenser and 5.0 g (38 mmol) gaseous TFAC was introduced into the reaction mixture. The reaction mixture was stirred at room temperature for 18 h. The resulting suspension was diluted with diethyl ether. After filtration, the volatiles were evaporated to yield the title compound as a solid 8.4 g (99 %). ¹HNMR : (500mhz, CDC13) ppm : 8.36 (bd, 2h), 7.46 (bd, 2h) ¹⁹FNMR : (471mhz, CDC13)ppm : -74.6 (s, 3F, CF3).

### Example 2 : Synthesis of 4-nitropentafluoroethoxybenzene

4-Nitrophenyl 2,2,2-trifluoroacetate (74 mmol, 17.4 g, crude product from example 1) was placed in a Hastelloy^{®} vessel and then 6.5 g HF (325 mmol), followed by 10 g SF₄ (94 mmol, 1.3 eq) were introduced into the vessel under dry ice cooling. The mixture was allowed to warm to room temperature and stirred at room temperature for 18 h. The pressure was 4 bar. The vessel was depressurized and all volatiles were removed under a light vacuum at 40°C to yield 18.3 g (96 %) of the title compound (99.6 % GC purity) as pale yellow crystals.
¹HNMR : (500mhz, CDC13) ppm : 8.38 (bd, 2h), 7.67 (bd, 2h)
¹⁹FNMR : (471mhz, CDC13) ppm : -85.4 (s, 3F, CF₃), -87.3 (s, 2F, CF₂).

## Claims

1. A process for the production of a compound of formula (I) R-CF₂-O-Ar wherein R is a fluorinated alkyl group or is an optionally substituted aromatic or heteroaromatic group and Ar is an optionally substituted aromatic or heteroaromatic group, comprising a step of reacting a compound of formula (II) R-C(O)-O-Ar wherein R and Ar are as defined above with a fluorination agent at a temperature of below +100°C.

2. The process of claim 1, wherein R is CF₃, CHF₂, CH₂F, CClF₂, CHClF or CCl₂F.

3. The process of claim 2, wherein R is CF₃.

4. The process of any one of claims 1-3, wherein Ar is optionally substituted phenyl.

5. The process of any one of claims 1-4, wherein Ar is substituted by an electron withdrawing group, preferably Ar is 4-NO₂-Ph.

6. The process of any one of claims 1-5, wherein the fluorination agent is a compound comprising a S-F-bond or a B-F-bond.

7. The process of claim 6, wherein the fluorination agent is SF₄.

8. The process of any one of the claims 1 to 7, wherein the process is conducted in the presence of a fluorination catalyst chosen from the group consisting of inorganic fluorides, preferably HF, AsF₃, PF₅ or TiF₄.

9. The process of claim 8, wherein the process is conducted in the presence of HF.

10. The process of claim 9, wherein the initial molar ratio HF : compound of formula (II) R-C(O)-O-Ar is from 2:1 to 6:1, preferably about 4:1.

11. The process of any one of the claims 1 to 10, wherein the reaction is carried out at a temperature of equal to or below 90°C, preferably at a temperature of equal to or below +60°C, more preferably at a temperature of equal to or below +40°C, and most preferably at a temperature of 0-25°C.

12. The process of any of claims 1-11, which comprises a step wherein the compound of formula (II) R-C(O)-O-Ar is manufactured by reacting a compound of formula (III) R-C(O)Cl with a compound of formula (IV) Ar-OH, wherein Ar and R are defined as in any of claim 1-11.

13. The process of claim 12, wherein the compound of formula (III) is trifluoro acetic acid chloride and the compound of formula (IV) is p-nitrophenol.

14. The process of claim 12 or 13, wherein the reaction of the compound of formula (III) with the compound of the formula (IV) Ar-OH is performed in the presence of an acid scavenger, wherein the molar ratio of acid scavenger to compound of the formula (III) is below 1.

15. The process of any of claims 12 to 14 wherein the product (II) is used in crude form in the fluorination reaction according to claims 1-11.
